# EUROPEAN PATENT SPECIFICATION

(11) **EP 1 496 760 B1**
(45) Date of publication and mention of the grant of the patent: **08.04.2009**
(21) Application number: 03728438.7
(22) Date of filing: 18.04.2003
(51) Int. Cl.: A23L 1/305, A23L 2/66, A23L 2/68, A23C 9/154, A23C 9/152, A23L 1/0524, A23L 1/053, A23L 1/308

(54) **ACIDIC COMPOSITIONS COMPRISING PROTEIN AND FIBER AND PROCESSES OF THEIR PREPARATION**
SAURE PROTEIN- UND BALLASTSTOFFHALTIGE ZUSAMMENSETZUNGEN UND VERFAHREN ZU IHRER HERSTELLUNG
COMPOSITIONS ACIDES COMPRENANT DES PROTEINES ET DES FIBRES ET PROCEDES DE PREPARATION

(30) Priority: 24.04.2002 US 376032 P
(43) Date of publication of application: 19.01.2005
(73) Proprietor: THE PROCTER & GAMBLE COMPANY, Cincinnati, Ohio 45202 (US)
(72) Inventor: NUNES, Raul, Victorino, Loveland, OH 45140 (US); KRUMMEN, Roger, William, Cincinnati, OH 45213 (US); SMALL, Leonard, Edwin, Cincinnati, OH 45229 (US); MEHANSHO, Haile, Fairfield, OH 45014 (US)
(74) Representative: Clemo, Nicholas Graham
(86) International application number: PCT/US2003/012046
(87) International publication number: WO 2003/090558

(56) References cited:
- EP-A- 0 765 609
- WO-A-02/00042
- WO-A-95/06417
- WO-A-99/56563
- US-A- 4 212 893
- US-A- 5 409 725
- US-A- 5 648 112
- US-A1- 2002 037 353
- US-B1- 6 207 638
- US-B1- 6 287 623
- GLAHN P E ET AL: "CASEIN-PECTIN INTERACTION IN SOUR MILK BEVERAGES" SYMPOSIUM ON FOOD INGREDIENTS EUROPE, XX, XX, 4 October 1994 (1994-10-04), pages 252-256, XP000604284

## Description

### FIELD OF THE INTENTION

The present invention relates to acidic compositions comprising protein and fiber. The compositions are particularly useful as food or beverage compositions.

### BACKGROUND OF THE INVENTION

Compositions containing milk proteins and fruit juice are well known Unfortunately, however, such compositions may be difficult to formulate since the acidic nature of the fruit juice can cause curdling and coagulation when combined with the milk protein. Accordingly, the milk protein and fruit juice components are not always compatible and present inherent challenges in forming a beverage composition that is physically stable and organoleptically desirable.

U.S. Patent No. 5,648,112, Yang et al., issued July 14, 1997, describes a method of producing a beverage composition that is physically stable and does not precipitate over time. The methods of Yang *et al*. are particularly beneficial for production of beverages containing fruit juice and protein. However, Yang *et al*. requires relatively low levels of stabilizer components, including certain fibrous materials. These required low levels may preclude delivery of nutritionally relevant amounts of fiber. Moreover, as described, the particulates must be prepared and maintained at a relatively small size (less than about 0.8 microns), which may present challenges with respect to practical manufacture of the composition. It would therefore be desirable to provide compositions and processes of their preparation which include protein components wherein nutritionally relevant amounts of fiber can be delivered under acidic conditions, while still allowing flexible manufacturing specifications (*e*.*g*., a more flexible range for particulate size).

US 6,287,623 teaches protein and fiber drinks having a pH of 2.5 to 5 and a particle size of less than 10 µm, such as 6.9 µm. WO 99/56563 describes low pH enteral formulae with preferred particle sizes of less than 0.5 µm. The formulae are beverages.

The present inventors have discovered that nutritionally relevant amounts of fiber may be included in such compositions, without compromising the stability or organoleptic properties of the composition. This result is particularly surprising, since increased fiber would be expected to induce undesired precipitation of various components within the composition (including not only the excess fiber, but other components as well). Even further, the inventors have discovered that the appropriate size range of particulates within the composition may be more flexible than previously reported, which can ease preparation of the compositions during the manufacturing process. Therefore, the present inventors have discovered acidic compositions and processes of their preparation that allow use of protein components without compromising stability, while also enabling other expanded parameters relative to those previously described.

### SUMMARY OF THE INVENTION

The present invention relates to acidic compositions comprising protein and fiber. The compositions comprise:
(a) a particulate protein component comprising whey, casein, and mixtures thereof;
(b) from 0.05% to 5% of a particulate fiber component, by weight of the composition; and
(c) a fatty acid material selected from fatty acids, non-glyceryl esters thereof, and mixtures thereof;
wherein the particles of fiber and protein have a median particle size of from greater than 0.8 microns to 1 micron and wherein the pH of the composition is from 3 to 5.

In yet another embodiment of the present invention, the compositions of the present invention are prepared by a process described herein. In particular, the compositions may be prepared by the following process:
(a) mixing the protein component and the fiber component to form a first mixture comprising the particles;
(b) mixing an acidic component and the first mixture at a temperature of from 4 °C to 43 °C to form a second mixture comprising the particles; and mixing a fatty acid material selected from fatty acids, non-glyceryl esters thereof, and mixtures thereof with the second mixture;
wherein the particles have a median particle size of from greater than 0.8 microns to 1 micron; and wherein the first mixture and second mixture are formed under high shear conditions.

### DETAILED DESCRIPTION OF THE INVENTION

All percentages and ratios are calculated by weight unless otherwise indicated. All percentages and ratios are calculated based on the total composition unless otherwise indicated.

Referenced herein are trade names for components including various ingredients utilized in the present invention. The inventors herein do not intend to be limited by materials under a certain trade name. Equivalent materials (*e*.*g*., those obtained from a different source under a different name or reference number) to those referenced by trade name may be substituted and utilized in the descriptions herein.

In the description of the invention various embodiments or individual features are disclosed. As will be apparent to the ordinarily skilled practitioner, all combinations of such embodiments and features are possible and can result in preferred executions of the present invention.

The compositions herein may comprise, consist essentially of, or consist of any of the elements as described herein.

### Definitions

As used herein, the term "NP/M" means net-power-per-unit-mass.

As used herein, the term "Watt/Kg" means watts per kilogram.

As used herein, all median particle sizes are defined in terms of Number Distribution. Number Distribution can be measured using a laser scattering system, *e*.*g*., *a* HORIBA LA910 Particle Size Analyzer (commercially available from Horiba, California).

### The Compositions of the Present Invention

The present compositions are beverages which are surprisingly stable and organoleptically appealing, despite the acidic nature of the compositions and the presence of both protein and fiber components.

The various components of the present compositions are described further as follows:

### The Protein Component

The protein component used herein comprises a protein selected from the group consisting of whey, casein, and mixtures thereof. The protein component may be the protein itself, for example as sodium or calcium caseinate, or may be a component comprising the protein and one or more other materials. For example, various milk proteins are known to one of ordinary skill in the art and may be utilized as the protein component herein.

Wherein the protein component is delivered as the protein itself, casein is the preferred protein for use herein. However, whey or mixtures of casein and whey may also be included. Casein may be utilized as a variety of forms including, for example, sodium or calcium caseinate. Mixtures of sodium and calcium caseinate are preferred for use herein.

Milk proteins include mammalian or vegetable milks such as, for example, whole milk, skim milk, condensed milk, dry milk powder, milk protein concentrate, milk protein isolate, milk protein hydrosylate, and mixtures thereof. To illustrate, milk protein concentrate is prepared *via* milk ultrafiltration or other means such that the lactose or ash content is reduced, thereby enhancing the protein content. In dry and condensed milk, water is removed but all other components of milk are substantially maintained. All forms of milk protein can comprise, for example, intact milk protein, milk protein hydrosylate, or any combination thereof.

The amount of protein component in the present compositions will depend upon a variety of factors including, for example, whether the protein component is the protein itself or delivered as a milk protein, the amount of protein desired in the final composition, and the like. Preferably, the compositions comprise at least 0.5% protein, by weight of the composition. More preferably, the compositions comprise from 0.5% to 10% protein, even more preferably from 0.5% to 8% protein, most preferably from 0.5% to 5% protein, and yet more preferably from 0.5% to 2% protein all by weight of the composition. Wherein the protein of the protein component is delivered via milk protein or another component, the amounts may be appropriately adjusted. For example, wherein the protein component is whole or skim milk, the compositions preferably comprise from about 5% to about 75%, more preferably from about 5% to about 40%, and most preferably from about 5% to about 15% of the protein component, all by weight of the composition. As another example, wherein the protein component is dry milk powder, the compositions preferably comprise from about 0.5% to about 10%, more preferably from about 0.5% to about 8%, and most preferably from about 0.5% to about 5% of the protein component, all by weight of the composition.

### The Fiber Component

The fiber component of the present invention comprises one or more fibrous materials. The fiber component, when used in the amounts indicated herein, has been found to stabilize the protein component under the acidic conditions of the composition. In particular, elevated amounts of fiber component may be utilized relative to amounts previously described, surprisingly without significant particle settling or precipitation. Such elevated amounts, of from 0.8% to 5% of fiber component (by weight of the composition), are important for use in compositions which are needed to deliver protein combined with nutritionally relevant levels of fiber.

The fiber components utilized herein include hydrophilic colloidal compounds commonly known in the art. See e.g., Yang et al., U.S. Patent No. 5,648,112, issued July 15, 1997. Non-limiting examples of such fiber components include gelatin, locust bean, cellulosic polymers (*e*.*g*., carboxymethylcellulose), pectins, psyllium, guar gum xanthan gum, alginates, gum arabic, fructo-oligosaccharides, inulin, agar, carrageenan, and mixtures thereof. Pectin, carboxymethylcelluose, gum arabic, or mixtures thereof are particularly preferred. In the most preferred embodiment herein, the fiber component is a mixture of pectin and gum arabic.

The compositions comprise 0.8% to 5%, more preferably from 0.9% to 2.5%, and most preferably from 1% to 2% of the fiber component, all by weight of the composition.

### The Median Particle Size

The present inventors have discovered that the allowable median particle size of the particulates present in the compositions herein may be up to and including about 1 micron, while still maintaining stabilized protein that exhibits little or no curdling or coagulation. This allowable median particle size is higher than previously reported, allowing more flexibility with regard to the parameters of the final compositions.

The particulates having this median particle size are those of the protein and fiber components, whereby the fiber component serves to stabilize and withhold the protein component from curdling or coagulation. Moreover, despite the relatively high levels of fiber component as set forth herein, little overall sedimentation or precipitation is observed.

The median particle size of the particles is therefore from greater than 0.8 microns to 1 micron. In addition, optionally, at least 90% of the particles have a particle size of less than 10 microns (more preferably less than 5 microns), most preferably at least 100% of the particles have a particle size of less than 10 microns (more preferably less than 5 microns).

### pH of the Compositions

The compositions of the present invention are acidic in nature. In particular, the present compositions have a pH of from 3 to 5, preferably from 3 to 4.5, more preferably from 3.5 to 4.5, and most preferably from 3.9 to 4.3.

Organic as well as inorganic edible acids may be used to adjust the pH of the beverage composition. The acids can be present in their undissociated form or, alternatively, as their respective salts, for example, potassium or sodium hydrogen phosphate, potassium or sodium dihydrogen phosphate salts. The preferred acids are edible organic acids including, for example, citric acid, malic acid, fumaric acid, adipic acid, phosphoric acid, gluconic acid, tartaric acid, ascorbic acid, acetic acid, phosphoric acid, or mixtures thereof. The most preferred acids are citric acid, malic acid, or mixtures thereof. Citric acid is particularly preferred for use herein.

### The Processes of the Present Invention

The compositions of the present invention may be prepared by the inventive processes described herein. In particular, the compositions may be prepared by the following process which comprises:
(a) mixing a protein component and a fiber component to form a first mixture comprising the particles; and
(b) mixing an acidic component and the first mixture at a temperature of from 4 °C to 43 °C to form a second mixture comprising the particles;
wherein the particles have the median particle size of from greater than 0.8 microns to 1 micron or less; and wherein the first mixture and second mixture are formed under high shear conditions. In a subsequent step, the fatty acid material is added.

The various steps of the present processes are described in more detail, as follows: Forming the First Mixture

The first mixture is formed by mixing the protein component and the fiber component. It is important to form this first mixture prior to substantial acidification. Otherwise, coagulation or curdling of the protein component is likely to occur. As has been discovered by the present inventors, the first mixture may comprise particles having a particle size of up to 1 micron
wherein the final composition comprises from 0.8% to 5% of the fiber component, by weight of the composition. As also discovered herein, relatively large particles are suitable, *i*.*e*., those having a particle size of from 0.9 microns to 1 micron. This is contrary to previous reports, which did not allow levels up to about 5% for a fibrous material, nor relatively large particles.

The first mixture is formed under high shear conditions. As used herein with respect to formation of the first mixture, the preferred high shear conditions are a NP/M of from about 5 Watt/Kg to about 75 Watt/Kg, more preferably from about 10 Watt/Kg to about 50 Watt/Kg, and most preferably from about 10 Watt/Kg to about 35 Watt/Kg. As will be understood by one of ordinary skill in the art, these are measures of mixing energy. Optional apparatuses for effecting this mixing energy are described separately below.

The first mixture may be formed at a variety of temperatures. Preferably, the first mixture is formed at a temperature of from 4 °C to 43 °C, more preferably from 4 °C to 30 °C, even more preferably from 10 °C to 22 °C, and most preferably from 14 °C to 22 °C. Means of temperature control are commonly known in the art.

The first mixture is preferably formed under aqueous conditions, such that the protein and fiber components are hydrated. In a particularly preferred embodiment of the present invention, the fiber component is added to water, followed by addition of the protein component.

Other optional components may be added during formation of the first mixture provided, however, that the mixture is substantially acidified subsequent to formation of the first mixture.

### Forming the Second Mixture

The second mixture is formed by mixing an acidic component and the first mixture. The acidic component will be readily understood by one of ordinary skill in the art. Organic as well as inorganic edible acids may be used to form the second mixture. The acids can be present in their undissociated form or, alternatively, as their respective salts, for example, potassium or sodium hydrogen phosphate, potassium or sodium dihydrogen phosphate salts. The preferred acids are edible organic acids including, for example, citric acid, malic acid, fumaric acid, adipic acid, phosphoric acid, gluconic acid, tartaric acid, ascorbic acid, acetic acid, phosphoric acid, or mixtures thereof. The most preferred acids are citric acid, malic acid, or mixtures thereof. The ordinarily skilled artisan will be able to determine the appropriate amount of added edible acid, depending upon the intended use of the finished composition.

The acidic component should be mixed with the first mixture slowly, for example, over a time period from about one minute to about one hour, preferably from about three minutes to about thirty minutes, all depending upon the specific batch size prepared. The present inventors have discovered that utilizing a slow addition rate results in surprisingly enhanced stability of the finished composition and is important for avoidance of curdling or coagulation.

The second mixture is formed under high shear conditions. As used herein with respect to forming the second mixture, the high shear conditions are a NP/M of from about 5 Watt/Kg to about 75 Watt/Kg, preferably from about 10 Watt/Kg to about 50 Watt/Kg, and most preferably from about 10 Watt/Kg to about 35 Watt/Kg. As will be understood by one of ordinary skill in the art, these are measures of mixing energy. Optional apparatuses for effecting this mixing energy are described separately below.

The second mixture is formed at a temperature of from 4 °C to 43 °C. Preferably, the second mixture is formed at 4 °C to 30 °C, even more preferably from 10 °C to 22 °C, and most preferably from 12 °C to 22 °C. Means of temperature control are commonly known in the art.

The second mixture is preferably formed under aqueous conditions.

Other optional components may added during formation of the first mixture provided, however, that the mixture is substantially acidified subsequent to formation of the first mixture The second mixture may be homogenized at a pressure of from 6.9 MPa (1,000 psi) to about 104 MPa (15,000 psi), more preferably at a pressure of from about 21 MPa (3,000 psi) to about 49 MPa (7,000 psi). Such homogenization may be conducted before and / or after any sterilization procedures, for example, under ultra-high temperature (UHT) conditions.

### Optional Mixtures

In one of the optional embodiments of the present processes, certain components such as minerals (*e*.*g*., calcium or other minerals described herein) may be present in the finished compositions. Preferably, wherein one or more such minerals is included, the present inventors have discovered that such components should be mixed with the other components of the composition subsequent to formation of the second mixture. Indeed, the present inventors have discovered that such subsequent mixing substantially avoids curdling or coagulation of the various components of the composition. In a particularly surprising finding, the inventors have discovered that minerals are preferably solubilized in an aqueous liquid (*e*.*g*., water) prior to mixing with the second mixture. Such pre-solubilization reduces the potential for reactivity with the protein or fiber components under the acidic conditions.

Formation of this optional mixture may be formed under low shear or high shear conditions. Preferably, the optional mixture is formed under high shear conditions such that the particles present in the composition maintain their defined particle size, as specified herein. As used with respect to formation of this optional mixture, the preferred high shear conditions are a NP/M of from about 10 Watt/Kg to about 75 Watt/Kg, more preferably from about 10 Watt/Kg to about 50 Watt/Kg, and most preferably from about 10 Watt/Kg to about 35 Watt/Kg. As will be understood by one of ordinary skill in the art, these are measures of mixing energy.

The optional mixture may be formed at a variety of temperatures. Preferably, the optional mixture is formed at a temperature of from about 4 °C to about 30 °C, more preferably from about 10 °C to about 22 °C, and most preferably from about 12 °C to about 22 °C. Means of temperature control are commonly known in the art.

The optional mixture may be homogenized at a pressure of from about 7 MPa (1,000 psi) to about 104 MPa (15,000 psi). Such homogenization may be conducted before and / or after any sterilization procedures, for example, under ultra-high temperature (UHT) conditions.

### Optional Mixing Apparatuses

As will be commonly understood, mixing energy is calculated through measure of the current and voltage used to deliver the energy to the components to form the dispersion. For example, a suitable power analyzer is available from Fluke Corporation, Everett, WA (e.g., Model 41B Power Harmonics Analyzer). Additionally, a suitable Amp Probe is Model 80I-1000S, also available from Fluke Corporation. Using these or similar instruments, the mixing energy is determined.

The means for subjecting the components to the mixing energy may be selected from a variety of well-known apparatuses (energizing means) that are commercially available. For example, this energizing means may be a mixer which provides energy to the liquid medium by forming ultrasonic vibrations therein, *e*.*g*., a Sonolator, commercially available from Sonic Corporation, Stratford, CT or Piezoelectric transducers. The Sonolator is an in-line system providing ultrasonic vibrations by pumping a liquid, a blend of liquids, or a solid dispersion in a liquid through a shaped orifice at a high linear velocity. The liquid stream impinges against a blade cantilevered in the stream. Flow over the blade causes vibrations in the blade that produces cavitation in the stream converting flow energy into mixing/dispersion energy. Other particularly useful energizing means include batch mixers providing a high agitator tip speed, e.g., blenders as available from Sunbeam Corporation of Delray Beach, FL with the brand name OSTERIZER. Additionally rotor/stator high shear mixers, commercially available from Charles Ross & Son, Hauppauge, NY may be useful. In-line mixers such as are available from Quadro Inc., Millburn, NJ, as model Quadro ZC/XC are useful as well. Additionally, particularly preferred energizing means for use herein include bottom-driven mixtures such as the Breddo Likwifier (Model LOR, round tank; Model LTD square tank) commercially available from Breddo Likwifier, Kansas City, MO and APV Mixer/Blender (Multiverter (round tank) / Liquiverter (square tank) high speed mixers Commercially available from APV Crepaco, Inc., Lake Mills, WI.

### Optional Components and Uses of the Present Compositions

The compositions of the present invention may comprise one or more additional optional components to, for example, enhance their performance or to otherwise render the composition more suitable for use as an industrial or consumer product. Accordingly, the present processes may optionally involve inclusion of one or more of these optional components. These components may be added to the compositions herein provided they do not substantially hinder important properties, particularly stabilization or organoleptic properties, of the compositions. Non-limiting examples of optional components are given below:

### Water

Water is typically included in the compositions of the present invention. As used herein, the term "water" includes the total amount of water present in the composition including from, for example, fluid milk or other milk protein source, fruit juice, or vegetable juice, as well as any added water. Water is preferably included at levels from about 10% to about 99.999%, more preferably from about 5% to about 99%, still more preferably at least about 50%, even more preferably at least about 70%, and most preferably from about 70% to about 99%, by weight of the composition. Ready-to-drink beverage compositions will typically comprise at least about 70% water, preferably from about 75% to about 99% water, all by weight of the composition. Ready-to-drink beverage compositions are particularly preferred.

### Flavor Agents

The compositions herein may optionally, but preferably, comprise one or more flavor agents. Preferably, such flavor agents are included in the beverage compositions and are typically selected from fruit juice, fruit flavors, botanical flavors, and mixtures thereof. Wherein fruit juice is included, the beverages of the present invention can comprise from about 0.1% to about 99%, preferably from about 1% to about 50%, more preferably from about 2% to about 30%, and most preferably from about 2% to about 20%, fruit juice. As measured herein, the weight percentage of fruit juice is based on a single strength 2° to 16° Brix fruit juice. The fruit juice can be incorporated into the beverage as a puree, comminute, or as a single strength or concentrated juice. Especially preferred is incorporation of the fruit juice as a concentrate with a solids content (primarily as sugar solids) of from about 20° to about 80° Brix.

The fruit juice can be any citrus juice, non-citrus juice, or mixture thereof, which are known for use in dilute juice beverages. The juice can be derived from, for example, apple, cranberry, pear, peach, plum, apricot, nectarine, grape, cherry, currant, raspberry, gooseberry, elderberry, blackberry, blueberry, strawberry, lemon, lime, mandarin, orange, grapefruit, cupuacu, potato, tomato, lettuce, celery, spinach, cabbage, watercress, dandelion, rhubarb, carrot, beet, cucumber, pineapple, coconut, pomegranate, kiwi, mango, papaya, banana, watermelon, passion fruit, tangerine, and cantaloupe. Preferred juices are derived from apple, pear, lemon, lime, mandarin, grapefruit, cranberry, orange, strawberry, tangerine, grape, kiwi, pineapple, passion fruit, mango, guava, raspberry and cherry. Citrus juices, preferably grapefruit, orange, lemon, lime, and mandarin juices, as well as juices derived from mango, apple, passion fruit, and guava, as well as mixtures of these juices are most preferred.

Fruit flavors may also be utilized, including flavors which mimic or are derived from any of the fruits described above. As described above with respect to flavor emulsions, fruit flavors may be derived from natural sources such as essential oil and extracts, or can be synthetically prepared. Fruit flavors may be derived from fruits through processing, particularly concentration. Wherein fruit juices are concentrated or evaporated, the condensate contains volatile substances that comprise the flavor of the fruit. Often, such flavor is added to a juice concentrate to enhance the flavor thereof.

Botanical flavors may also be utilized. As used herein, the term "botanical flavor" refers to a flavor derived from parts of a plant other than the fruit; *i*.*e*., derived from nuts, bark, roots, and / or leaves. Also included within the term "botanical flavor" are synthetically prepared flavors made to simulate botanical flavors derived from natural sources. Botanical flavors can be derived from natural sources such as essential oils and extracts, or can be synthetically prepared. Suitable botanical flavors include chocolate, vanilla, jamaica, kola, marigold, chrysanthemum, chamomile, ginger, valerian, yohimbe, hops, eriodictyon, ginseng, bilberry, rice, red wine, mango, peony, lemon balm, nut gall, oak chip, lavender, walnut, gentiam, luo han guo, cinnamon, angelica, aloe, agrimony, yarrow and mixtures thereof. Particularly preferred flavors include chocolate or vanilla.

Wherein coffee solids are included, the compositions typically comprise from about 3% to about 23% coffee solids, by weight of the composition. Wherein tea solids are included, the compositions of the present invention can comprise from about 0.01% to about 1.2%, preferably from about 0.05% to about 0.8%, by weight of the composition, of tea solids. The term "tea solids" as used herein means solids extracted from tea materials including those materials obtained from the genus *Camellia* including *C*. *sinensis* and *C. assaimica*, for instance, freshly gathered tea leaves, fresh green tea leaves that are dried immediately after gathering, fresh green tea leaves that have been heat treated before drying to inactivate any enzymes present, unfermented tea, instant green tea, and partially fermented tea leaves. Green tea solids are tea leaves, tea plant stems, and other plant materials that are related and which have not undergone substantial fermentation to create black teas. Members of the genus *Phyllanthus, Catechu gambir* and Uncaria family of tea plants can also be used.

### Sweeteners

The compositions of the present invention can contain an effective amount of one or more sweeteners, including carbohydrate sweeteners and natural or artificial no/low calorie sweeteners.

The compositions of the present invention can be sweetened with any of the carbohydrate sweeteners, preferably monosaccharides and / or disaccharides. Sweetened compositions, particularly beverages, will typically comprise from about 0.1% to about 40%, more preferably from about 0.1 % to about 20%, and most preferably from about 1% to about 8%, sweetener, all by weight of the composition. These sweeteners can be incorporated into the compositions in solid or liquid form but are typically, and preferably, incorporated as a syrup, most preferably as a concentrated syrup such as high fructose corn syrup. For purposes of preparing beverages of the present invention, these sugar sweeteners can be provided to some extent by other components of the beverage such as, for example, the fruit juice component and / or flavors.

Preferred sugar sweeteners for use in compositions of the present invention are sucrose, fructose, glucose, and mixtures thereof. Fructose can be obtained or provided as liquid fructose, high fructose corn syrup, dry fructose or fructose syrup. Other naturally occurring sweeteners or their purified extracts, such as glycyrrhizin, the protein sweetener thaumatin, the juice of Luo Han Guo disclosed in, for example, U.S. Patent No. 5,433,965, and the like can also be used in the compositions of the present invention.

Suitable no/low calorie sweeteners include, for example, saccharin, cyclamates, L-aspartyl-L-phenylalanine lower alkyl ester sweeteners (*e*.*g*., aspartame); L-aspartyl-D-alanine amides disclosed in Brennan *et al*., U.S. Patent No. 4,411,925; L-aspartyl-D-serine amides disclosed in U.S. Patent 4,399,163; L-aspartyl-L-1-hydroxymethylalkaneamide sweeteners disclosed in U.S. Patent No. 4,338,346; L-aspartyl-1-hydroxyethyalkaneamide sweeteners disclosed in U.S. Patent No. 4,423,029; L-aspartyl-D-phenylglycine ester and amide sweeteners disclosed in European Patent Application 168,112, published January 15, 1986; N-[N-3,3-dimethylbutyl)-L-alpha-aspartyl]-L-phenylalanine 1-methyl ester sweeteners disclosed in WO 99/30576, assigned to The Nutrasweet Co., published June 24, 1999; alltame, thaumatin; dihydrochalcones; cyclamates; steviosides; glycyrrhizins, synthetic alkoxy aromatics; sucralose; suosan; miraculin; monellin; sorbitol, xylitol; talin; cyclohexylsulfamates; substituted imidazolines; synthetic sulfamic acids such as acesulfame, acesulfame-K and n-substituted sulfamic acids; oximes such as perilartine; peptides such as aspartyl malonates and succanilic acids; dipeptides; amino acid based sweeteners such as gem-diaminoalkanes, *meta*-aminobenzoic acid, L-aminodicarboxylic acid alkanes, and amides of certain alpha-aminodicarboxylic acids and gem-diamines; and 3-hydroxy-4-alkyloxyphenyl aliphatic carboxylates or heterocyclic aromatic carboxylates; erythritol; and mixtures thereof.

### Coloring Agent

Coloring agents may be utilized in the compositions of the present invention. For example, natural or artificial colors may be used.

FD&C dyes (*e*.*g*., yellow #5, blue #2, red # 40) and / or FD&C lakes are preferably used. By adding the lakes to the other powdered ingredients, all the particles, in particular the colored iron compound, are completely and uniformly colored and a uniformly colored composition is attained. Preferred lake dyes which may be used in the present invention are the FDA-approved Lake, such as Lake red #40, yellow #6, blue #1, and the like. Additionally, a mixture of FD&C dyes or a FD&C lake dye in combination with other conventional food and food colorants may be used.

Other coloring agents, for example, natural agents may be utilized. Non-limiting examples of such other coloring agents include fruit and vegetable juices, riboflavin, carotenoids (for example, beta-carotene), tumeric, and lycopenes.

The exact amount of coloring agent used will vary, depending on the agents used and the intensity desired in the finished composition. Generally, if utilized, the coloring agent is typically present at a level of from about 0.0001% to about 0.5%, preferably from about 0.001% to about 0.1%, and most preferably from about 0.004% to about 0.1%, by weight of the composition.

### Nutrients

The compositions herein can be fortified with one or more nutrients, defined herein as one or more vitamins and / or minerals. Indeed, the present inventors have discovered that nutrients, particularly minerals, may be added to the present compositions without compromising the stability of the composition. This is particularly surprising, as addition of minerals such as calcium would be expected to cause coagulation or curdling in the composition.

Unless otherwise specified herein, wherein a given mineral is present in the composition, the composition comprises at least about 1%, preferably at least about 5%, more preferably from about 10% to about 200%, even more preferably from about 40% to about 150%, and most preferably from about 60% to about 125% of the USRDI of such mineral. Unless otherwise specified herein, wherein a given vitamin is present in the composition, the composition comprises at least about 1%, preferably at least about 5%, more preferably from about 10% to about 200%, even more preferably from about 20% to about 150%, and most preferably from about 25% to about 120% of the USRDI of such vitamin. The U.S. Recommended Daily Intake (USRDI) for vitamins and minerals are defmed and set forth in the Recommended Daily Dietary Allowance-Food and Nutrition Board, National Academy of Sciences-National Research Council.

Non-limiting examples of such vitamins and minerals include iron, zinc, copper, phosphorous, biotin, folic acid, pantothenic acid, iodine, vitamin A, vitamin C, vitamin B₁, vitamin B₂, vitamin B₃, vitamin B₆, vitamin B₁₂, vitamin D, vitamin E, and vitamin K. Preferably, wherein a vitamin or mineral is utilized the vitamin or mineral is selected from iron, zinc, folic acid, iodine, vitamin A, vitamin C, vitamin B₁, vitamin B₃, vitamin B₆, vitamin B₁₂, vitamin D, and vitamin E.

Commercially available vitamin A sources may also be included in the present compositions. As used herein, "vitamin A" includes, but is not limited to, retinol, β-carotene, retinol palmitate, and retinol acetate. The vitamin A may be in the form of, for example, an oil, beadlets or encapsulated.

Wherein vitamin A is present in the compositions herein, the composition comprises at least about 1%, preferably at least about 5%, more preferably from about 10% to about 200%, even more preferably from about 15% to about 150%, and most preferably from about 20% to about 120% of the USRDI of vitamin A. The quantity of vitamin A to be added is dependent on processing conditions and the amount of vitamin A deliver desired after storage. Preferably, wherein vitamin A is included within the present compositions, the compositions comprise from about 0.0001% to about 0.2%, more preferably from about 0.0002% to about 0.12%, also preferably from about 0.0003% to about 0.1%, even more preferably from about 0.0005% to about 0.08%, and most preferably from about 0.001% to about 0.06% of vitamin A, by weight of the composition.

Commercially available sources of vitamin B₂ (also known as riboflavin) may be utilized in the present compositions. Wherein vitamin B₂ is present in the compositions herein, the composition comprises at least about 1%, preferably at least about 5%, more preferably from about 5% to about 200%, even more preferably from about 10% to about 100%, and most preferably from about 10% to about 50% of the USRDI of vitamin B₂.

Commercially available sources of vitamin C can be used herein. Encapsulated ascorbic acid and edible salts of ascorbic acid can also be used. Wherein vitamin C is present in the compositions herein, the composition comprises at least about 1%, preferably at least about 5%, more preferably from about 10% to about 200%, even more preferably from about 20% to about 150%, and most preferably from about 25% to about 120% of the USRDI of such vitamin.

The quantity of vitamin C to be added is dependent on processing conditions and the amount of vitamin C deliver desired after storage. Preferably, wherein vitamin C is included within the present compositions, the compositions comprise from about 0.005% to about 0.2%, more preferably from about 0.01% to about 0.12%, also preferably from about 0.02% to about 0.1%, even more preferably from about 0.02% to about 0.08%, and most preferably from about 0.03% to about 0.06% of vitamin C, by weight of the composition.

Nutritionally supplemental amounts of other vitamins which may be incorporated herein include, but are not limited to, biotin, vitamins B₁, B₃, B₆ and B₁₂, folic acid, pantothenic acid, folic acid, vitamin D, and vitamin E. Wherein the composition comprises one of these vitamins, the composition preferably comprises at least 5%, preferably at least 25%, and most preferably at least 35% of the USRDI for such vitamin.

Minerals which may optionally be included in the composition herein are, for example, magnesium, zinc, iodine, iron, and copper. As an example, any soluble salt of these minerals suitable for inclusion in edible compositions can be used, for example, magnesium citrate, magnesium gluconate, magnesium sulfate, zinc chloride, zinc sulfate, potassium iodide, copper sulfate, copper gluconate, and copper citrate.

Calcium is a particularly preferred mineral for use in the present invention. Preferred sources of calcium include, for example, amino acid chelated calcium, calcium carbonate, calcium oxide, calcium hydroxide, calcium sulfate, calcium chloride, calcium phosphate, calcium hydrogen phosphate, calcium dihydrogen phosphate, calcium citrate, calcium malate, calcium titrate, calcium gluconate, calcium propionate, tricalcium phosphate, and calcium lactate, and in particular calcium citrate-malate.

The form of calcium citrate-malate is described in, *e*.*g*., U.S. Patent Nos. 5,670,344; 5,612,026; 5,571,441; 5,474,793; 5,468,506; 5,445,837; 5,424,082; 5,422,128; 5,401,524; 5,389,387; 5,314,919; 5,232,709; 5,225,221; 5,215,769; 5,186,965; 5,151,274; 5,128,374; 5,118,513; 5,108,761; 4,994,283; 4,786,510; and 4,737,375.

Wherein calcium is included, the compositions will typically comprise from about 0.01% to about 0.5%, more preferably from about 0.03% to about 0.2%, even more preferably from about 0.05% to about 0.15%, and most preferably from about 0.1% to about 0.15% of calcium, all by weight of the composition.

Iron may be utilized in the compositions of the present invention. Acceptable forms of iron are well-known in the art. The amount of iron compound incorporated into the composition will vary widely depending upon the level of supplementation desired in the final composition and the targeted consumer. Iron fortified compositions of the present invention typically contain from about 5% to about 100%, preferably from about 15% to about 50%, and most preferably about 20% to about 40% of the USRDI for iron.

Highly bioavailable ferrous salts that can be used in the ingestible compositions of the present invention are ferrous sulfate, ferrous fumarate, ferrous succinate, ferrous gluconate, ferrous lactate, ferrous tartarate, ferrous citrate, ferrous amino acid chelates, as well as mixtures of these ferrous salts. While ferrous iron is typically more bioavailable, certain ferric salts can also provide highly bioavailable sources of iron.

Certain ferric salts can also provide highly bioavailable sources of iron. Highly bioavailable ferric salts that can be used in the food or beverage compositions of the present invention are ferric saccharate, ferric ammonium citrate, ferric citrate, ferric sulfate, ferric pyrophosphate, ferric orthophosphate, as well as mixtures of these ferric salts. Combinations or mixtures of highly bioavailable ferrous and ferric salts can be used.

A particularly preferred ferric iron source is ferric pyrophosphate, for example, microencapsulated SUNACTIVE Iron, commercially available from Taiyo International, Inc., Edina, Minnesota, U.S.A and Yokkaichi, Mie, Japan. SUNACTIVE Iron is particularly preferred for use herein due to its particle size, compatibility, and bioavailability.

Ferrous amino acid chelates particularly suitable as highly bioavailable iron sources for use in the present invention are those having a ligand to metal ratio of at least 2:1. For example, suitable ferrous amino acid chelates having a ligand to metal mole ratio of two are those of formula:

Fe(L)₂

where L is an alpha amino acid, dipeptide, tripeptide, or quadrapeptide ligand. See e.g., U.S. Patent Nos. 4,863,898; 4,830,716; and 4,599,152. Particularly preferred ferrous amino acid chelates are those where the reacting ligands are glycine, lysine, and leucine. Most preferred is the ferrous amino acid chelate sold under the mark FERROCHEL (Albion Laboratories, Salt Lake City, Utah) wherein the ligand is glycine.

Other sources of iron particularly suitable for fortifying compositions of the present invention included certain iron-sugar-carboxylate complexes. In these iron-sugar-carboxylate complexes, the carboxylate provides the counterion for the ferrous or ferric iron. These iron-sugar-carboxylate complexes can be prepared in the manner described in, *e*.*g*., Nakel et al., U.S. Patent Nos. 4,786,510 and 4,786,518, issued November 22, 1988. These materials are referred to as "complexes", but they may exist in solution as complicated, highly hydrated, protected colloids; the term "complex" is used for the purpose of simplicity.

Zinc may also be utilized in the compositions of the present invention. Acceptable forms of zinc are well-known in the art. Zinc fortified compositions of the present invention typically contain from about 5% to about 100%, preferably from about 15% to about 50%, and most preferably about 25% to about 45% of the USRDI for zinc. The zinc compounds which can be used in the present invention can be in any of the commonly used forms such as, *e*.*g*., zinc sulfate, zinc chloride, zinc acetate, zinc gluconate, zinc ascorbate, zinc citrate, zinc aspartate, zinc picolinate, amino acid chelated zinc, and zinc oxide. Zinc gluconate and amino acid chelated zinc are particularly preferred.

### Edible Carriers

One or more edible carriers may be utilized in the present compositions. In particular, for example wherein one or more nutrients is included within the compositions, the edible carrier comprises an emulsifier such as that described in Mehansho et a/., U.S. Patent No. 5,888,563, issued March 30, 1999. Such edible carriers may reduce undesirable flavors and colors associated with such nutrients. A preferred edible carrier for use herein is lecithin. Lecithin may be commercially obtained, for example, as soy lecithin (commercially available from Central Soya, Fort Wayne, IN).

### Fatty Acid Materials

The compositions comprise a fatty acid material. The fatty acid material is selected from fatty acids, non-glyceryl esters thereof, and mixtures thereof. As used herein, the fatty acid material contains a fatty acid chain, or wherein the fatty acid material is a fatty acid ester, contains a fatty acid chain and an ester chain. Thus, wherein the fatty acid material is a fatty acid, the material is depicted as follows:

R-COOH

wherein "R" is the fatty acid chain which is a saturated or unsaturated chain having at least 9 carbon atoms, typically from 9 to 25 carbon atoms, and wherein "COOH" is a carboxylic acid moiety. More preferably, "R" is a saturated or unsaturated chain having from about 11 to about 23, preferably from about 15 to about 21 carbon atoms and, depending upon the embodiment herein, often preferably from about 15 to about 17 carbon atoms. Also preferably, the fatty acid chain contains from 0 to about 3 double bonds. Most preferably, the fatty acid chain is unsaturated, in particular having one or two double bonds.

Wherein the fatty acid material is a non-glyceryl ester of a fatty acid *(i*.*e*., a "non-glyceryl ester thereof"), the material is depicted as follows:

R-COOR'

Wherein R is the fatty acid chain as defined above, and R' is the ester chain, with the carboxylate moiety "COO" linking the two together. The ester chain is a straight or branched chain of carbon atoms which is hydrolyzable in the presence of mammalian digestive enzymes, preferably human digestive enzymes, and typically contains no more than about 8 carbon atoms. The ester chain more preferably contains from 1 to about 5 carbon atoms and, again, may be a straight (for example, n-propyl) or branched (for example, *iso*-propyl) chain. Highly preferred ester chains include those which form methyl esters (i.e., R' is -CH₃), ethyl esters, *n*-propyl esters, *iso*-propyl esters, *n*-butyl esters, *iso*-butyl esters, and mixtures thereof. Those ester chains which form ethyl esters are particularly preferred.

In a preferred embodiment of the present invention, the fatty acid material is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, eicosapentaenoic acid, behenic acid, erucic acid, docosahexaenoic acid, lignoceric acid, non-glyceryl esters thereof, and mixtures thereof. Preferred non-glyceryl esters of fatty acids include ethyl oleate, ethyl linoleate, and mixtures thereof.

In a particularly preferred embodiment of the present invention, the fatty acid material is selected from lauric acid, lauroleic acid, myristic acid, myristoleic acid, pentadecanoic acid, palmitic acid, palmitoleic acid, margaric acid, stearic acid, dihydroxystearic acid, oleic acid, ricinoleic acid, elaidic acid, linoleic acid, alpha-linolenic acid, dihomogamma-linolenic acid, eleostearic acid, licanic acid, arachidonic acid, arachidic acid, eicosenoic acid, behenic acid, erucic acid, lignoceric acid, non-glyceryl esters thereof, and mixtures thereof. In this embodiment of the invention, it is particularly preferred to select a fatty acid material containing from 0 to about 3 double bonds and having a fatty acid chain length of from about 15 to about 17 carbon atoms. Additionally, particularly preferred fatty acid materials include oleic acid, linoleic acid, esters thereof, and mixtures thereof. Preferred non-glyceryl esters of this embodiment include ethyl oleate, ethyl linoleate, and mixtures thereof. As an example, ethyl oleate may be obtained from a variety of sources, including Victorian Chemical Co., Richmond, Victoria Australia; Penta Manufacturing Co., Livingston, NJ; and Croda, Inc., Parsippany, NJ.

In another preferred embodiment of the present invention, the fatty acid material is selected from omega-3-fatty acids, non-glyceryl esters thereof, and mixtures thereof. The omega-3-fatty acids are particularly preferred for use herein due to their beneficial effects on the health of the consumer, particularly in the fields of skin and cardiac health.

As is well-understood in the art, and as consistently used herein, the term "omega-3-fatty acid" is utilized to refer to those fatty acid materials having an omega-3 double bond wherein the omega-3 double bond is positioned between the third and fourth carbon atoms of the fatty acid chain when counting from the omega (distal) carbon atom of the chain. Omega-3-fatty acids are preferably derived from marine (fish) sources, including menhaden (a herring-like fish). Non-limiting examples of preferred omega-3-fatty acid sources include OMEGAPURE, commercially available from Omega Protein, Inc., Houston, TX.

Non-limiting examples of omega-3-fatty acids which are suitable for use herein include eicosapentaenoic acid (also known as EPA), docosahexaenoic acid (also known as DHA), and mixtures thereof. Non-glyceryl esters thereof are also contemplated.

The compositions comprise from about 0.0001% to about 10% of the fatty acid material, by weight of the composition, and depending upon the particular embodiment desired (for example, a concentrate suitable for further dilution or a ready-to-drink beverage composition). More preferably, the compositions comprise from about 0.01% to about 5% of the fatty acid material, by weight of the composition. Even more preferably, the compositions comprise from about 0.01% to about 3% of the fatty acid material, by weight of the composition. Most preferably, the compositions comprise from about 0.5% to about 2.5% of the fatty acid material, by weight of the composition. For example, a typical composition of the present invention may comprise from about 1% to about 1.3% of the fatty acid material, by weight of the composition.

### Carbonation Component

Carbon dioxide can be introduced into the water which is mixed with a beverage syrup or into the dilute beverage after dilution to achieve carbonation. The carbonated beverage can be placed into a container, such as a bottle or can, and then sealed. Any conventional carbonation methodology may be utilized to make carbonated beverage compositions of this invention. The amount of carbon dioxide introduced into the beverage will depend upon the particular flavor system utilized and the amount of carbonation desired.

### EXAMPLES

The following are non-limiting examples of the present compositions and processes. The following examples are provided to illustrate the invention and are not intended to limit the scope thereof in any manner.

### Example 1

A citrus-flavored beverage is prepared having the following components in the indicated amounts:

| **Component** | **Weight Percent** |
|---|---|
| Sodium Citrate | 0.06 |
| Pectin | 0.3 |
| Gum Arabic | 1.5 |
| Sodium Caseinate | 0.55 |
| Calcium Caseinate | 1.18 |
| Sugar | 1.02 |
| Citric Acid | 0.28 |
| Acesulfame K | 0.01 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 2

A citrus-flavored beverage is prepared having the following components in the indicated amounts:

| **Component** | **Weight Percent** |
|---|---|
| Sodium Citrate | 0.06 |
| Pectin | 0.3 |
| Gum Arabic | 1.5 |
| Sodium Caseinate | 0.55 |
| Calcium Caseinate | 1.18 |
| Sugar | 1 |
| Citric Acid | 0.28 |
| Acesulfame K | 0.01 |
| Sucralose, 25% solution | 0.04 |
| Calcium Propionate | 0.03 |
| Ethyl Oleate | 0.6 |
| Emulsifier (Steroyl Lactylate, *e*.*g*., EMPLEX K, commercially Available from American Ingredients, Inc.) | 0.05 |
| Calcium Lactate | 0.04 |
| Erythritol | 1.97 |
| Flavoring Agents (including mango, peach, and vanilla flavors) | 1.55 |
| Water | *Quantum satis* |

### Example 3

A citrus-flavored beverage is prepared having the following components in the indicated amounts:

| **Component** | **Weight Percent** |
|---|---|
| Sodium Citrate | 0.06 |
| Pectin | 0.6 |
| Gum Arabic | 1.5 |
| Sodium Caseinate | 0.55 |
| Calcium Caseinate | 1.18 |
| Sugar | 1.02 |
| Citric Acid | 0.28 |
| Acesulfame K | 0.01 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 4

A citrus-flavored beverage is prepared having the following components in the indicated amounts:

| **Component** | **Weight Percent** |
|---|---|
| Sodium Citrate | 0.06 |
| Pectin | 0.3 |
| Gum Arabic | 1.5 |
| Sodium Caseinate | 1 |
| Calcium Caseinate | 2.3 |
| Sugar | 1.02 |
| Citric Acid | 0.28 |
| Acesulfame K | 0.01 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 5

A citrus-flavored beverage is prepared having the following components in the indicated amounts:

| **Component** | **Weight Percent** |
|---|---|
| Sodium Citrate | 0.06 |
| Pectin | 0.6 |
| Gum Arabic | 1.5 |
| Sodium Caseinate | 1 |
| Calcium Caseinate | 1.75 |
| Sugar | 1.02 |
| Citric Acid | 0.28 |
| Acesulfame K | 0.006 |
| Sucralose, 25% solution | 0.04 |
| Calcium Propionate | 0.03 |
| Ethyl Oleate | 0.61 |
| Calcium Lactate | 0.04 |
| Erythritol | 1.97 |
| Flavoring Agents (including mango, peach, and vanilla flavors) | 1.55 |
| Water | *Quantum satis* |

### Example 6

A composition according to any of the preceding examples is prepared according to the following process, which is performed at a substantially constant temperature of about 20 °C:

A Likwifier and mixing tank are provided, wherein the Likwifier is positioned to run in circulation with the mixing tank. Water is added to the Likwifier. As part of forming the first mixture, the sodium citrate, pectin, and gum arabic are added to the Likwifier in sequence under conditions of high shear for about four minutes (depending upon batch size). To finalize formation of the first mixture, the sodium caseinate, calcium caseinate, and sugar are added to the Likwifier in sequence under conditions of high shear for about four minutes, whereby the caseinates become hydrated. To form the second mixture, the citric acid is slowly added to the first mixture under conditions of high shear over a time period of about three to about thirty minutes, depending upon batch size. The acesulfame K and sucralose solution are added to the second mixture under high shear conditions for about four minutes. Circulation with the mixing tank is ceased. A pre-mixture of the calcium lactate, calcium propionate, and water is formed and added to the resulting mixture in the mixing tank under low shear conditions. The ethyl oleate (fatty acid material) is then added to the resulting mixture under low shear conditions. The remaining components are then added under low shear conditions. The composition may optionally be homogenized at about 5,000 psi, sterilized under ultra-high temperature (UHT) conditions, homogenized at 3,500 psi, and aseptically packed.

## Claims

1. A beverage composition comprising:
a) a particulate protein component comprising whey, casein, or mixtures thereof;
b) from 0.8% to 5% by weight of a particulate fiber component; and
c) a fatty acid material selected from fatty acids, non-glyceryl esters thereof, and mixtures thereof;
wherein the pH of the composition is from 3 to 5 and the particles of fiber and protein have a median particle size of from greater than 0.8 microns to 1 micron.

2. The composition according to Claim 1 having a pH of from 3 to 4.5.

3. The composition according to any of the preceding claims comprising from 1% to 2% of the fiber component, by weight of the composition.

4. The composition according to any of the preceding claims comprising from 0.5% to 2% of the protein, by weight of the composition.

5. The composition according to any of the preceding claims further comprising a mineral.

6. The composition according to Claim 5 wherein the fatty acid material is selected from oleic acid, linoleic acid, non-glyceryl esters thereof, and mixtures thereof.

7. A process for preparing a composition according to any of the preceding claims comprising:
a) mixing the protein component and the fiber component to form a first mixture comprising the particles;
b) mixing an acidic component with the first mixture at a temperature of from 4°C to 43°C to form a second mixture comprising the particles; and
c) mixing a fatty acid material selected from fatty acids, non-glyceryl esters thereof, and mixtures thereof with the second mixture;
wherein the particles have a median particle size of from greater than 0.8 microns to 1 micron; and wherein the first mixture and second mixture are formed under high shear conditions.

8. The process according to Claim 7 wherein the first mixture is prepared by a method comprising:
a) mixing the fiber component under the high shear conditions to form a fiber mixture; and
b) adding the protein component to the fiber mixture and further mixing under the high shear conditions.

9. The process according to Claim 7 wherein the composition further comprises one or more minerals and the process comprises the step of solubilising the minerals in water before adding them to the second mixture.

## Patentansprüche

1. Getränkezusammensetzung, umfassend:
a) einen teilchenförmigen Proteinbestandteil, der Molke, Casein oder Mischungen davon umfasst;
b) zu 0,8 Gew.-% bis 5 Gew.-% einen teilchenförmigen Faserbestandteil; und
c) ein Fettsäurematerial, ausgewählt aus Fettsäuren, Nicht-Glycerylestern davon und Mischungen davon;
wobei der pH-Wert der Zusammensetzung 3 bis 5 beträgt und die Teilchen aus Faser und Protein eine mittlere Teilchengröße von mehr als 0,8 Mikrometer bis 1 Mikrometer aufweisen.

2. Zusammensetzung nach Anspruch 1, die einen pH-Wert von 3 bis 4,5 aufweist.

3. Zusammensetzung nach einem der vorstehenden Ansprüche, die zu 1 Gew.-% bis 2 Gew.-% der Zusammensetzung den Faserbestandteil umfasst.

4. Zusammensetzung nach einem der vorstehenden Ansprüche, die zu 0,5 Gew.-% bis 2 Gew.-% der Zusammensetzung das Protein umfasst.

5. Zusammensetzung nach einem der vorstehenden Ansprüche, die ferner ein Mineral umfasst.

6. Zusammensetzung nach Anspruch 5, wobei das Fettsäurematerial aus Oleinsäure, Linolsäure, Nicht-Glycerylestern davon und Mischungen davon ausgewählt ist.

7. Verfahren zum Herstellen einer Zusammensetzung nach einem der vorstehenden Ansprüche, umfassend:
a) Mischen des Proteinbestandteils und des Faserbestandteils, um eine erste Mischung zu bilden, die die Teilchen umfasst;
b) Mischen eines saueren Bestandteils mit der ersten Mischung bei einer Temperatur von 4 °C bis 43 °C, um eine zweite Mischung zu bilden, die die Teilchen umfasst; und
c) Mischen eines Fettsäurematerials, ausgewählt aus Fettsäuren, Nicht-Glycerylestern davon und Mischungen davon, mit der zweiten Mischung;
wobei die Teilchen eine mittlere Teilchengröße von mehr als 0,8 Mikrometer bis 1 Mikrometer aufweisen; und wobei die erste Mischung und die zweite Mischung unter Hochscherbedingungen gebildet werden.

8. Verfahren nach Anspruch 7, wobei die erste Mischung durch ein Verfahren hergestellt wird, das Folgendes umfasst:
a) Mischen des Faserbestandteils unter den Hochscherbedingungen, um eine Fasermischung zu bilden; und
b) Zugeben des Proteinbestandteils zu der Fasermischung und weiteres Mischen unter den Hochscherbedingungen.

9. Verfahren nach Anspruch 7, wobei die Zusammensetzung ferner einen oder mehrere Mineralstoffe umfasst und das Verfahren den Schritt des Löslichmachens der Mineralien in Wasser vor deren Zugabe zu der zweiten Mischung umfasst.

## Revendications

1. Composition de boisson comprenant :
a) un composant de protéine particulaire comprenant du petit-lait, de la caséine, ou leurs mélanges ;
b) de 0,8 % à 5 % en poids d'un composant de fibre particulaire ; et
c) une matériau de type acide gras choisi parmi les acides gras, les esters non glycéryliques de ceux-ci et leurs mélanges ;
dans laquelle le pH de la composition va de 3 à 5 et les particules de fibre et de protéine ont une taille moyenne de particules allant de plus de 0,8 micron à 1 micron.

2. Composition selon la revendication 1 ayant un pH allant de 3 à 4,5.

3. Composition selon l'une quelconque des revendications précédentes comprenant de 1 % à 2 % du composant de fibre, en poids de la composition.

4. Composition selon l'une quelconque des revendications précédentes comprenant de 0,5 % à 2 % de la protéine, en poids de la composition.

5. Composition selon l'une quelconque des revendications précédentes comprenant, en outre, un minéral.

6. Composition selon la revendication 5, dans laquelle le matériau de type acide gras est choisi parmi l'acide oléique, l'acide linoléique, les esters non glycéryliques de ceux-ci et leurs mélanges.

7. Procédé pour préparer une composition selon l'une quelconque des revendications précédentes comprenant :
a) un mélange du composant de protéine et du composant de fibre de façon à former un premier mélange comprenant les particules ;
b) un mélange d'un composant acide avec le premier mélange à une température allant de 4 °C à 43 °C de façon à former un deuxième mélange comprenant les particules ; et
c) un mélange d'un matériau de type acide gras choisi parmi les acides gras, leurs esters non glycériques, et leurs mélanges avec le deuxième mélange ;
dans lequel les particules ont une taille moyenne de particules allant de plus de 0,8 micron à 1 micron ; et dans lequel le premier mélange et le deuxième mélange sont formés dans des conditions de cisaillement élevé.

8. Procédé selon la revendication 7, dans lequel le premier mélange est préparé par un procédé comprenant :
a) un mélange du composant de fibre dans les conditions de cisaillement élevé de façon à former un mélange de fibres ; et
b) un ajout du composant de protéine au mélange de fibres et un mélange supplémentaire dans les conditions de cisaillement élevé.

9. Procédé selon la revendication 7, dans lequel la composition comprend en outre un ou plusieurs minéraux et le procédé comprend l'étape consistant à solubiliser les minéraux dans de l'eau avant de les ajouter au deuxième mélange.
